# DEMANDE DE BREVET EUROPEEN

(11) **EP 1 870 076 A2**
(43) Date de publication de la demande: **26.12.2007**
(21) Numéro de dépôt: 07291088.8
(22) Date de dépôt: 21.06.2001
(51) Int. Cl.: A61K 8/06, A61K 8/88, A61Q 1/02

(54) **Emulsion solide à phase grasse liquide structurée par un polymère**

(30) Priorité: 23.06.2000 FR 0008084
(62) Demande divisionnaire de: 01947570.6
(71) Demandeur: L'Oréal, 75008 Paris (FR)
(72) Inventeur: Lemann, Patricia, 26740 Montboucher sur Jabron (FR)
(74) Mandataire: Chantraine, Sylvie Hélène

(57) **Abrégé**

L'invention se rapporte à une émulsion solide physiologiquement acceptable, notamment cosmétique, contenant une phase aqueuse et une phase grasse liquide dispersées, l'une dans l'autre, la dite phase grasse liquide étant structurée par au moins un polymère de masse moléculaire moyenne en poids allant de 1000 à 30 000, comportant a) un squelette polymérique, ayant des motifs de répétition hydrocarbonés pourvus d'au moins un hétéroatome, et b) des chaînes grasses pendantes et/ou terminales éventuellement fonctionnalisées, ayant de 12 à 120 atomes de carbone et étant liées à ces motifs, la phase aqueuse, la phase grasse liquide et le polymère formant un milieu physiologiquement acceptable. Cette composition se présente notamment sous forme de fond de teint coulé, dont l'application conduit à un dépôt naturel, léger, frais et de bonne tenue dans le temps.

## Description

La présente invention se rapporte à une composition de soin et/ou de traitement et/ou de maquillage de la peau, y compris du cuir chevelu, et/ou des lèvres des êtres humains de type émulsion, contenant une phase grasse liquide structurée par un polymère particulier. Cette composition se présente notamment sous forme d'un produit de maquillage en particulier coulé en stick ou en coupelle et plus spécialement de stick anti-cerne, de maquillage du corps, de fond de teint ou de produit à lèvres, dont l'application conduit à un dépôt naturel, léger et de bonne tenue dans le temps. Plus spécialement, cette composition est sous forme d'émulsion solide eau-dans-huile (E/H).

Dans les produits cosmétiques ou dermatologiques solides, il est courant de trouver une phase grasse liquide structurée, à savoir gélifiée et/ou rigidifiée à l'aide de cires et/ou de charges ; ceci est notamment le cas dans les compositions solides comme les déodorants, les baumes et les rouges à lèvres, les produits anti-cerne, les fards à paupière et les fonds de teint coulés en pot ou en coupelle. Les produits coulés en coupelle sont souvent appelés des compacts.

Par "phase grasse liquide", au sens de l'invention, on entend une phase grasse liquide à température ambiante (25°C) et pression atmosphère (760mm de Hg), composée d'un ou plusieurs composés non aqueux liquides à température ambiante, appelés aussi huiles, généralement compatibles entre eux et insolubles au moins en partie dans une phase aqueuse. Les composés non aqueux ou huiles peuvent être volatils ou non volatils. Ainsi, la phase grasse liquide peut contenir un ou plusieurs composés volatils, un ou plusieurs composés non volatils ou un mélange de ces composés volatils et non volatils.

Par "composé volatil", on entend au sens de l'invention tout milieu non aqueux susceptible de s'évaporer au contact de la peau ou des lèvres en moins d'une heure, à température ambiante et pression atmosphérique. Le ou les solvants volatils de l'invention sont des solvants organiques et notamment des huiles cosmétiques volatiles, liquides à température ambiante, ayant une pression de vapeur non nulle, à température ambiante et pression atmosphérique, allant en particulier de 10⁻³ à 300mm de Hg et de préférence supérieure à 0,3mm de Hg.

La structuration de la phase grasse liquide permet en particulier de limiter son exsudation des compositions solides notamment dans les régions chaudes et humides et, en plus, de limiter, après dépôt sur la peau ou les lèvres, la migration ou propagation de cette phase dans les rides et ridules, ce qui est particulièrement recherché pour un rouge à lèvres, un produit anti-cerne ou un fard à paupières. Par "migration", on entend un débordement de la phase grasse liquide en dehors du tracé initial du maquillage. Cette migration de la phase grasse liquide, en particulier lorsqu'elle est importante et que cette phase est chargée de matières colorantes, conduit à un effet inesthétique autour des lèvres et des yeux, accentuant particulièrement les rides et les ridules. Cette migration est souvent citée par les femmes comme un défaut majeur des produits de maquillage colorés comme les rouges à lèvres, produits anti-cerne et fard à paupières classiques.

De plus, les compositions de maquillage ou de soin coulées se présentent le plus souvent sous forme anhydre entraînant, en particulier sur le visage et le corps mais aussi sur les cils, des phénomènes d'inconfort, de lourdeur, de gras et parfois des impressions de masque ou d'étouffement, qui peuvent devenir rédhibitoires.

Pour alléger le maquillage et réduire ces phénomènes de lourdeur et de gras, les cosméticiens se sont intéressés aux compositions de fond de teint et de rouges à lèvres sous forme d'émulsion. Ainsi, la société Shiseido a envisagé dans sa demande de brevet EP-A-0 374 332 des compositions de rouge à lèvres et de fond de teint, produisant un effet frais, sous forme d'émulsion solide du type eau-dans-huile contenant une huile de silicone volatile, une cire solide hydrocarbonée, un organopolysiloxane modifié par un groupe polyoxyalkylène, servant d'émulsionnant de la phase aqueuse dans la phase grasse, et des charges pulvérulentes. De même la société Procter & Gamble à décrit dans le document US-A-5 688 831 des compositions de maquillage hydratantes comportant une ou plusieurs silicones volatiles associées à un ou plusieurs humectants, des pigments et un composé amphiphile organique capable de former sur la peau ou dans la composition des cristaux liquides lyotropes smectiques renfermant lesdits humectants.

Ces compositions, du fait de la présence d'un taux élevé de cire présentent encore l'inconvénient d'être lourdes, d'être difficiles à étaler et de conférer le plus souvent à la composition un toucher désagréable. De plus, la présence de ces cires provoque généralement une matification du maquillage pas toujours souhaitable pour un rouge à lèvres ou un fard à paupières.

Il subsiste donc le besoin d'une composition ne présentant pas les inconvénients ci-dessus, et ayant notamment des propriétés de fraîcheur, de légèreté, de bonne tenue dans le temps, ne desséchant pas la peau ou les lèvres sur lesquelles elle est appliquée, aussi bien lors de l'application qu'au cours du temps. De plus, cette composition est stable dans le temps et facile à fabriquer. Le maquillage obtenu est naturel, sans sensation de masque ni d'étouffement et homogène.

L'invention a justement pour objet une émulsion solide de soin et/ou de maquillage et/ou de traitement de la peau et/ou des lèvres du visage et/ou des phanères permettant de remédier aux inconvénients mentionnés ci-dessus.

Par "émulsion", on entend une composition contenant une phase aqueuse et une phase grasse liquide dont l'une des deux phases est dispersée dans l'autre phase avec ou sans émulsionnant, l'ensemble étant homogène à oeil nu. Par "émulsion solide", on entend une émulsion qui ne s'écoule pas sous son propre poids à température ambiante et pression atmosphérique.

De façon surprenante, le demandeur a trouvé que l'utilisation de polymères particuliers dans une émulsion solide contenant une phase aqueuse et une phase grasse permettait l'obtention d'un produit coulé ou compact dont l'application sur la peau, les lèvres ou les fibres kératiniques conduisait à un film ayant des propriétés cosmétiques remarquables. En particulier, le film est, souple, confortable et "frais". De plus, la composition (ou l'émulsion) est stable dans le temps et n'exsude pas à température ambiante.

Par stable, on entend une composition qui n'exsude pas à température ambiante pendant au moins 2 moins, voire jusqu'à 9 mois.

L'invention s'applique non seulement aux produits de maquillage des lèvres, comme les rouges à lèvres, les brillants à lèvres et les crayons à lèvres et de maquillage de la peau, aussi bien du visage que du corps humain, comme les fonds de teints éventuellement coulés en stick, en pot ou en coupelle, les produits anti-cerne, les fards à paupières ou à joues et les produits de tatouage éphémère mais aussi aux produits de soin et/ou de traitement de la peau, y compris du cuir chevelu, et des lèvres comme les produits notamment en stick de protection solaire de la peau du visage ou des lèvres, aux produits d'hygiène corporelle comme les déodorants notamment en stick, les shampooings et après- shampooings épaissis et aux produits de maquillage des yeux comme les eye-liners, les crayons et les mascaras plus spécialement coulés sous forme de pain, ainsi qu'aux produits de maquillage ou de soin des fibres kératiniques comme les cheveux et les sourcils.

De façon plus précise, l'invention a pour objet une émulsion solide contenant une phase aqueuse et une grasse liquide dispersées l'une dans l'autre, la phase grasse liquide étant structurée par au moins un polymère de masse moléculaire moyenne en poids allant de 1000 à 30 000, comportant a) un squelette polymérique ayant des motifs de répétition hydrocarbonés pourvus d'au moins un hétéroatome, et b) des chaînes grasses pendantes et/ou terminales éventuellement fonctionnalisées, ayant de 12 à 120 atomes de carbone et étant liées à ces motifs hydrocarbonés, la phase aqueuse, la phase grasse liquide et le polymère formant un milieu physiologiquement acceptable.

La composition de l'invention peut se présenter sous forme de pâte ou de solide, plus ou moins dur, déformable ou non. Elle peut être une émulsion huile-dans-eau (H/E) ou eau-dans-huile (E/H), voire même sous forme d'émulsion triple. En particulier, elle se présente sous forme coulée en stick ou en coupelle et plus spécialement sous forme d'une émulsion eau-dans-huile rigide notamment coulée.

La structuration de la phase grasse liquide est modulable selon la nature du polymère à hétéroatome utilisé et peut être telle que l'on obtienne une structure plus ou moins dure notamment sous forme d'un bâton ou d'un stick. Ces compositions rigides lorsqu'elles sont colorées permettent, après application, d'obtenir un dépôt, homogène en couleur plus ou moins brillant, frais, non lourd, souple, ne migrant pas et de bonne tenue notamment de la couleur dans le temps.

Le polymère structurant de la composition de l'invention est un solide non déformable à température ambiante (25°C) et pression atmosphérique (760mm de Hg). Il est capable de structurer la composition sans l'opacifier.

Par "chaînes fonctionnalisées" au sens de l'invention, on entend une chaîne alkyle comportant un ou plusieurs groupes fonctionnels ou réactifs notamment choisis parmi les groupes hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Par "polymère", on entend au sens de l'invention un composé ayant au moins 2 motifs de répétition.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes non pendants et se trouvant dans le squelette polymérique. Ces hétéroatomes sont choisis parmi les atomes d'azote, de soufre, de phosphore et leurs associations, associés éventuellement à un ou plusieurs atome d'oxygène. Ces motifs peuvent, en outre, comprendre un groupement polaire du type carbonyle.

Ces motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide. Avantageusement, les chaînes pendantes sont liées directement à l'un au moins des hétéroatomes du squelette polymérique.

Entre, les motifs hydrocarbonés, le polymère peut comprendre des motifs siliconés ou des motifs oxyalkylénés.

En outre, le polymère de la composition de l'invention comprend avantageusement de 40 à 98 % de chaînes grasses par rapport au nombre total des motifs à hétéroatome et des chaînes grasses et mieux de 50 à 95 %. La nature et la proportion des motifs à hétéroatome est fonction de la nature de la phase grasse liquide et est en particulier similaire à la nature de la phase grasse. Ainsi, plus les motifs à hétéroatome sont polaires et en proportion élevée dans le polymère, ce qui correspond à la présence de plusieurs hétéroatomes, plus le polymère a de l'affinité avec les huiles polaires. En revanche, plus les motifs à hétéroatome sont peu polaires voire apolaires ou en proportion faible, plus le polymère a de l'affinité avec les huiles apolaires.

L'invention a aussi pour objet une émulsion solide contenant une phase aqueuse et une phase grasse liquide dispersées l'une dans l'autre, la phase grasse liquide étant structurée par au moins un polyamide de masse moléculaire moyenne en poids allant de 1000 à 30 000, comportant a) un squelette polymérique, ayant des motifs répétitifs amide, et b) éventuellement des chaînes grasses pendantes et/ou terminales éventuellement fonctionnalisées, ayant de 12 à 120 atomes de carbone et étant liées à ces motifs amide, la phase aqueuse, la phase grasse liquide et le polymère formant un milieu physiologiquement acceptable.

Cette émulsion peut être utilisée telle quelle ou introduite dans une composition plus complexe à milieu physiologiquement acceptable.

De préférence, les chaînes grasses pendantes sont liées à l'un au moins des atomes d'azote des motifs amide.

En particulier, les chaînes grasses de ce polyamide représentent de 40 à 98 % du nombre total des motifs amide et des chaînes grasses, et mieux de 50 à 95 %.

Comme polymères structurant préférés utilisables dans l'invention, on peut citer les polyamides ramifiés par des chaînes grasses pendantes et/ou des chaînes grasses terminales ayant de 12 à 120 atomes de carbone et notamment de 12 à 68 atomes de carbone, les chaînes grasses terminales étant liées au squelette polyamide par des groupes ester. Ces polymères sont plus spécialement ceux décrits dans le document US-A-5783657 de la société Union Camp. Chacun de ces polymères satisfait notamment à la formule (I) suivante : 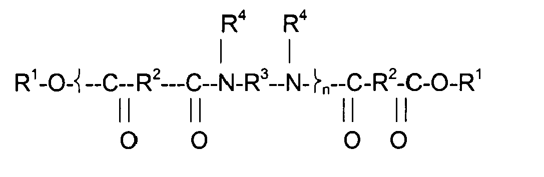 dans laquelle n désigne un nombre entier de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone et notamment de 4 à 24 atomes de carbone ; R² représente à chaque occurrence indépendamment un groupe hydrocarboné saturé ou non en C₄ à C₄₂ à condition que 50 % des groupes R² représentent un groupe hydrocarboné saturé ou non en C₃₀ à C₄₂; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle saturé ou non en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5. De préférence, R¹ est un groupe alkyle saturé ou non en C₁₂ à C₂₂ et de préférence en C₁₆ à C₂₂. Avantageusement, R² peut être un groupe hydrocarboné (alkylène saturé ou insaturé) en C₁₀ à C₄₂. De préférence, 50 % au moins et mieux 75 % des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂ saturé ou non. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés.

De façon avantageuse, le polymère de la composition de l'invention comprend une masse moléculaire moyenne en poids allant de 2 000 à 20 000 et mieux de 2 000 à 10 000.

Selon l'invention, la structuration de la phase grasse liquide est obtenue à l'aide d'un ou plusieurs polymères de formule (I). En général, les polymères de formule (I) se présentent sous forme de mélanges de polymères, ces mélanges pouvant en outre contenir un produit de synthèse de formule (I) correspondant à un composé avec n valant 0, c'est-à-dire un diester.

A titre d'exemple de polymères structurant utilisables dans la composition selon l'invention, on peut citer les produits commerciaux fabriqués et/ou vendus par la société Bush Boake Allen sous les noms Uniclear 80 et Uniclear 100. De tels produtis commerciaux sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylstéarylique.

Comme polymère structurant utilisable dans l'invention, on peut encore citer les résines polyamides résultant de la condensation d'un acide di-carboxylique aliphatique et d'une diamine (incluant les composés ayant respectivement plus de 2 groupes carboxyle et plus de 2 groupes amine), les groupes carboxyle et amine de motifs unitaires adjacents étant condensés sous forme d'une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiliser les polyamides vendus par la société Union Camp Corp. sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Les polymères structurant de la composition de l'invention ont avantageusement une température de ramollissement supérieure à 70°C et pouvant aller jusqu'à 190°C. De préférence, il présente une température de ramollissement allant de 80 à 130°C. Ces polymères sont en particulier des polymères non cireux.

Avantageusement, le polymère est associé à au moins un composé amphiphile liquide et non volatile à température ambiante, de valeur de balance hydrophile/lipophile (HLB) inférieure à 12 et notamment allant de 1 à 8 et de préférence de 1 à 5. Selon l'invention, on peut utiliser un ou plusieurs composés amphiphiles. Ces composés amphiphiles ont pour but de renforcer les propriétés structurantes du polymère à hétéroatome, de faciliter la mise en oeuvre du polymère et d'améliorer la capacité à déposer de l'émulsion solide.

Selon l'invention, la composition de type d'émulsion peut avoir une dureté allant de 10g à 200g et mieux de 30g à 100g. Cette dureté peut être mesurée selon une méthode de pénétration d'une sonde dans ladite composition et en particulier à l'aide d'un analyseur de texture (par exempleTA-XT2 de chez Rhéo) équipé d'un cylindre en ébonite de 5 mm de haut et 8 mm de diamètre. La mesure de dureté est effectuée à 20°C au centre de 5 échantillons de la dite composition. Le cylindre est introduit dans chaque échantillon de composition à une pré-vitesse de 2mm/s puis à une vitesse de 0,5 mm/s et enfin à une post-vitesse de 2mm/s, le déplacement total étant de 1 mm. La valeur relevée de la dureté est celle du pic maximum. L'échantillon a une épaisseur d'environ 1 cm.

La dureté de la composition (ou de l'émulsion) selon l'invention est telle que la composition est autoportée et peut se déliter aisément pour former un dépôt satisfaisant sur la peau et/ou les lèvres et/ou les phanères. En outre, avec cette dureté, la composition de l'invention résiste bien aux chocs.

Selon l'invention, l'émulsion solide notamment coulée en coupelle ou éventuellement sous forme de stick a le comportement d'un solide élastique déformable et souple, conférant à l'application une douceur élastique remarquable. Les compositions coulées de l'art antérieur n'ont pas cette propriété d'élasticité et de souplesse.

Le ou les composés amphiphiles utilisables dans la composition de l'invention comprennent une partie lipophile liée à une partie polaire, la partie lipophile comportant une chaîne carbonée ayant au moins 8 atomes de carbone notamment, de 18 à 32 atomes de carbone et mieux de 18 à 28 atomes de carbone. De préférence, la partie polaire de ce ou ces composés amphiphiles est le reste d'un composé choisi parmi les alcools et les polyols ayant de 1 à 12 groupements hydroxyle, les polyoxyalkylènes comportant au moins 2 motifs oxyalkylénés et ayant de 0 à 20 motifs oxypropylénés et/ou de 0 à 20 motifs oxyéthylénés. En particulier, le composé amphiphile est un ester choisi parmi les hydro-xystéarates, les oléates, les iso-stéarates du glycérol, du sorbitan ou du méthylglucose ou encore les alcools gras ramifiés en C₁₂ à C₂₆ comme l'octyldodécanol et leurs mélanges. Parmi ces esters, on préfère les monoesters et les mélanges de mono- et de di-esters.

Le taux de composé amphiphile et celui du polymère à hétéroatome sont choisis selon la dureté de gel désirée et en fonction de l'application particulière envisagée. Les quantités respectives de polymère et de composé amphiphile doivent être telles qu'elles permettent l'obtention d'un stick délitable. En pratique, la quantité de polymère (en matière active) représente de 0,5 à 80 % du poids total de la composition et mieux de 5 à 40 %, voire de 7 à 20%. La quantité de composé amphiphile représente en pratique de 0,1 % à 35 % du poids total de la composition et mieux de 1 % à 15 %, s'il est présent.

Avantageusement, la phase grasse liquide de la composition contient au moins 40 % d'huile(s) liquide(s) ayant un groupement similaire à celui des motifs à hétéroatome et mieux de 50 à 100 %. En particulier, la phase grasse liquide structurée par un squelette de type polyamide contient une quantité majoritaire, à savoir au moins égale à 40 % du poids total de la phase grasse liquide et mieux de 50 à 100 %, d'huile ou mélange d'huiles liquides apolaires, et plus spécialement d'huile(s) hydrocarbonée(s).

Pour une phase grasse liquide structurée par un polymère comportant un squelette en partie siliconée, cette phase grasse contient de préférence au moins 40% du poids total de la phase grasse liquide et mieux de 50 à 100 %, d'huile ou mélange d'huiles liquides siliconées, par rapport au poids total de la phase grasse liquide.

Pour une phase grasse liquide structurée par un polymère apolaire du type hydrocarboné, cette phase grasse contient avantageusement au moins 40 % en poids et mieux de 50 à 100 %, d'huile ou mélange d'huiles apolaires liquides, notamment hydrocarbonées, par rapport au poids total de la phase grasse liquide.

Par "huile hydrocarbonée", on entend toute huile contenant des atomes de carbone et d'hydrogène et éventuellement une fonction éther, ester, acide ou hydroxyle.

En particulier, les huiles polaires de l'invention sont :
- les huiles végétales hydrocarbonées à forte teneur en triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les huiles de synthèse ou esters de synthèse de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), l'isononanoate d'isononyle, le benzoate d'alcool en C₁₂ à C₁₅, le myristate d'isopropyle, le palmitate d'éthyl 2-hexyle, l'isostéarate d'isostéarate, des octanoates, décanoates ou ricinoléates d'alcools ou de polyalcools ; les esters hydroxylés comme le lactate d'isostéaryle, le malate de di-isostéaryle ; et les esters du pentaérythritol ;
- les éthers de synthèse ayant de 10 à 40 atomes de carbone ;
- les alcools gras en C₈ à C₂₆ comme l'alcool oléique ;
- leurs mélanges.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle, alcoxy ou phényle, pendant et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, des diphényl diméthicones, les diphényl méthyldiphényl trisiloxanes, les 2-phényléthyl triméthylsiloxysilicates ; les hydrocarbures linéaires ou ramifiés d'origine synthétique ou minérale, volatils ou non, comme les huiles de paraffine, volatiles ou non volatiles, et ses dérivés, la vaseline, la lanoline liquide, les polydécènes, le polyisobutène hydrogéné tel que le parléam, le squalane ; et leurs mélanges. De préférence, les huiles structurées, et plus spécialement celles structurées par les polyamides et en particulier ceux de formules (I) ou les polyuréthanes ou les polyurées ou les polyurée-uréthanes, sont des huiles apolaires et plus spécialement une huile ou un mélange d'huiles du type hydrocarboné d'origine minérale ou synthétique, choisies en particulier parmi les hydrocarbures notamment les alcanes comme l'huile de parléam, les isoparaffines comme l'isododécane et le squalane et leurs mélanges. Avantageusement, ces huiles sont associées à une ou plusieurs huiles de silicones phénylées.

De préférence, la phase grasse liquide contient, au moins une huile non volatile choisie en particulier parmi les huiles hydrocarbonées d'origine minérale, végétale ou synthétique, les esters ou éthers de synthèse, les huiles de silicone et leurs mélanges.

La phase grasse liquide totale représente, en pratique, de 5 à 98 % du poids total de la composition, de préférence de 20 à 75 %.

La phase grasse liquide de la composition selon l'invention peut contenir, en outre, au moins un composé non aqueux volatil, à savoir un ou plusieurs composés volatils. Les composés volatils sont en général des solvants des composés non volatils.

Selon l'invention, ces composés volatils facilitent, notamment, l'application de la composition sur la peau, les lèvres ou les phanères. Ces composés volatils peuvent être des composés hydrocarbonés, des composés siliconés comportant éventuellement des groupements alkyle ou alkoxy pendants ou en bout de chaîne siliconée ou un mélange de ces composés volatils.

Comme composé volatil utilisable dans l'invention, on peut citer les huiles de silicones linéaires ou cycliques ayant une viscosité à température ambiante inférieure à 10 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Comme autre composé volatil utilisable dans l'invention, on peut citer les huiles volatiles hydrocarbonées ayant de 8 à 16 atomes de carbone et leurs mélanges et notamment les alcanes ramifiés en C₈-C₁₆ comme les iso-alcanes (appelées aussi isoparaffines) en C₈-C₁₆, l'isododécane, l'isodécane, l'isohexadécane et par exemple les huiles vendues sous les noms commerciaux d'Isopars' ou de Permetyls, les esters ramifiés en C₈-C₁₆ comme le néopentanoate d'iso-hexyle et leurs mélanges. De préférence, le composé (ou solvant) volatil est choisi parmi les huiles volatiles hydrocarbonées ayant de 8 à 16 atomes de carbone et leurs mélanges.

On peut aussi utiliser des composés volatils fluorés.

Ces composés volatils représentent notamment un taux massique de 0 à 97,5 % par rapport au poids total de la composition, de préférence de 1 à 75 % et mieux de 3 à 45 % (si présents). De façon générale, la quantité de composé volatil est utilisée en une quantité suffisante pour obtenir des propriétés de glissant et d'étalement.. Cette quantité sera adaptée par l'homme du métier en fonction de l'intensité des propriétés d'étalement recherchée.

La composition de l'invention contient, en outre, une phase aqueuse non miscible à la phase grasse liquide contenant de l'eau éventuellement épaissie ou gélifiée par un ou plusieurs épaississants ou gélifiants de phase aqueuse et contenant éventuellement des composés miscibles à l'eau, comme des alcools inférieurs en C₂ à C₇, les polyols ayant au moins deux groupements hydroxyle et de 2 à 10 atomes de carbone comme le glycérol, le propylène glycol, de D-panthénol, les polyéthylènes glycols. La phase aqueuse représente notamment de 1 à 80 % du poids total de la composition et mieux de 5 à 70%.

L'émulsion selon l'invention peut être obtenue en utilisant un tensioactif ou un mélange de tensioactifs dont le HLB (balance hydrophile/lipophile) est adapté au sens de l'émulsion.

Comme tensioactif utilisable dans l'invention, adapté à l'obtention d'une émulsion E/H on peut citer ceux ayant un HLB inférieur 7 et notamment les ester d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone. Comme tensioactif utilisable dans l'invention pour l'obtention d'une émulsion H/E on peut citer ceux ayant un HLB supérieur à 7 comme les esters d'acides gras de polyéthylène glycol (monostéarate ou monolaurate de polyéthylène glycol) ; les esters d'acides gras (stéarate, oléate) de sorbitol polyoxyéthylénés ; les alkyl (lauryl, cétyl, stéaryl, octyl) éthers polyoxyéthylénés et les diméthicones copolyols. De façon générale, on peut utiliser tout tensioactif ionique (cationique ou anionique) amphotère et tout tensioactif non ionique, bien connu de l'homme du métier.

La composition de l'invention peut comprendre, en outre, tout additif usuellement utilisé dans le domaine concerné, choisi notamment parmi les matières colorantes, les antioxydants, les huiles essentielles, les conservateurs, les parfums, les électrolytes (cation ou polycations), les charges, les cires, les produits pâteux à température ambiante, les neutralisants, les polymères liposolubles ou dispersibles dans le milieu, les actifs cosmétiques ou dermatologiques comme par exemple des émollients, des hydratants, des vitamines, des acides gras essentiels, des filtres solaires, les dispersants comme l'acide poly(12-hydroxystéarique) et leurs mélanges. Ces additifs peuvent être présents dans la composition à raison de 0 à 20% (notamment de 0,01 à 20 %) du poids total de la composition et mieux de 0,01 à 10%. Avantageusement, la composition contient au moins un actif cosmétique ou dermatologique.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut se présenter sous la forme d'une composition teintée dermatologique ou de soin des matières kératiniques comme la peau, les lèvres et/ou les phanères tels que les fibres kératiniques, sous forme d'une composition de protection solaire ou d'hygiène corporelle notamment sous forme de produit déodorant ou démaquillant sous forme coulée. Elle peut notamment être utilisée comme base de soin pour la peau, les phanères ou les lèvres (baumes à lèvres protégeant les lèvres du froid et/ou du soleil et/ou du vent, produit de soin pour la peau, les ongles ou les cheveux).

La composition de l'invention peut également se présenter sous la forme d'un produit coloré de maquillage de la peau, en particulier un fond de teint, présentant éventuellement des propriétés de soin ou de traitement, un blush, un fard à joues ou à paupières, un produit anti-cerne, un eye-liner, un produit de maquillage du corps ; de maquillage des lèvres comme un rouge à lèvres, un brillant à lèvres ou un crayon présentant éventuellement des propriétés de soin ou de traitement ; de maquillage des phanères comme les cils et les cheveux en particulier sous forme d'un mascara pain, les sourcils notamment sous forme de crayon, les ongles.

Bien entendu la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains. Par cosmétiquement acceptable, on entend au sens de l'invention une composition d'aspect, d'odeur et de toucher agréables.

Avantageusement, la composition contient au moins un actif cosmétique et/ou un actif dermatologique et/ou au moins une matière colorante. Grâce à l'utilisation d'au moins un polymère de masse moléculaire moyenne en poids allant de 1000 à 30 000, tels que défini précédemment dans une émulsion, on obtient un piégeage des actifs et des matières colorantes présents dans la composition, permettant de les maintenir la où ils ont été appliqués, à savoir les lèvres, la peau ou les phanères.

La matière colorante selon l'invention peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 50 % du poids total de la composition, de préférence de 5 à 30 %, si elle est présente.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0,1 à 20 % du poids de la compositions et mieux de 0,1 à 6 % (si présents).

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0,1 à 50 % et mieux de 2 à 30 % du poids total de la composition, s'ils sont présents.

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0,1 à 20 % du poids total de la composition et mieux de 0,1 à 15 %, s'ils sont présents.

La composition peut éventuellement contenir une ou plusieurs cires pour améliorer la structuration sous forme de stick, bien que cette forme rigide puisse être obtenue en l'absence de cire. Une cire, au sens de la présente invention, est un composé gras lipophile, solide à température ambiante (25°C), à changement d'état solide/liquide réversible, ayant une température de fusion supérieure à 40°C pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange. C'est cette recristallisation dans le mélange qui est responsable de la diminution de la brillance dudit mélange. Aussi, avantageusement la composition contient peu ou pas de cire.

Les cires, au sens de la demande, sont celles généralement utilisées dans les domaines cosmétique et dermatologique ; elles sont notamment d'origine naturelle comme la cire d'abeilles, la cire de Carnauba, de Candellila, d'Ouricoury, du Japon, de fibres de liège ou de canne à sucre, les cires de paraffine, de lignite, les cires microcristallines, la cire de lanoline, la cire de Montan, les ozokérites, les huiles hydrogénées comme l'huile de jojoba hydrogénée, mais aussi d'origine synthétique comme les cires de polyéthylène issues de la polymérisation de l'éthylène, les cires obtenues par synthèse de Fischer-Tropsch, les esters d'acides gras et les glycérides concrets à 40°C, les cires de silicone comme les alkyle, alcoxy et/ou esters de poly(di)méthylsiloxane solide à 40°C.

La composition selon l'invention peut être fabriquée par les procédés connus, généralement utilisés dans le domaine cosmétique ou dermatologique. Elle peut être fabriquée par le procédé qui consiste à chauffer le polymère au moins à sa température de ramollissement, à y ajouter le ou les composés amphiphiles, les émulsionnants, les matières colorantes et les additifs éventuellement fondus puis à mélanger le tout jusqu'à l'obtention d'une solution claire, transparente. On ajoute alors, au mélange obtenu, après abaissement de la température, le ou les composés volatils, puis la phase aqueuse. L'ensemble est ensuite homogénéisé. Le mélange homogène obtenu peut alors être coulé dans un moule approprié comme une coupelle ou un pot de brillant à lèvres ou directement dans les articles de conditionnement (boîtier ou coupelle notamment).

L'invention a encore pour objet un procédé cosmétique de soin, de maquillage ou de traitement des matières kératiniques des êtres humains et notamment de la peau, des lèvres et des phanères, comprenant l'application sur les matières kératiniques de la composition notamment cosmétique telle que définie ci-dessus.

L'invention est illustrée plus en détail dans les exemples suivants. Les pourcentages sont donnés en poids.

### Exemple 1 : Fond de teint E/H

### Phase A

- Isostéarate de sorbitane (Arlacel 987d'Uniquema) 4,5 %
- Conservateur 0,2 %

### Phase B

- Uniclear 80 15 %
- Octyl-2-dodécanol 3,6 %
- Iso-paraffine hydrogénée (parléam) 11 %

### Phase C

- Dioxyde de titane 4,1 %
- Iso-paraffine hydrogénée 7 %
- Oxyde de fer 1,3 %

### Phase D

- Parfum 0,65 %

### Phase E

.Eau qsp 100 %
Conservateur 0,3 %
. Sulfate de magnésium 0,7 %
Propylène glycol 3 %

La phase (B) est chauffée à 120°C, sous agitation, puis homogénéisée à l'aide d'un homogénéisateur Rainery tournant à 300 trs/min. On ajoute ensuite la phase A puis la phase C prébroyée à l'aide d'un broyeur tri-cylindre. On ajoute ensuite doucement et régulièrement la phase D et la phase E au mélange précédent ramené à 100°C avec un homogénéïsateur Heidolph. L'ensemble est laissé en contact pendant 10 min puis coulé dans des coupelles.

Le produit obtenu est solide, de prise au doigt facile ; il est frais et s'étale bien et forme sur la peau un dépôt homogène, léger. Le maquillage obtenu est naturel et satiné. Sa dureté en g (au texturomètre) est de 35.

### Exemple 2 : Fond de teint solide E/H

### Phase A

Isostéarate de sorbitane (Arlacel 987d'Uniquema) 4,5 %
Conservateur 0,2 %

### Phase B

Uniclear 80 15 %
Octyl-2-dodécanol 3,6 %
. Iso-paraffine hydrogénée (parléam) 6 %
Isododécane 5 %

### Phase C

- Dioxyde de titane 4,1 %
- Iso-paraffine hydrogénée 7 %
- Oxyde de fer 1,3 %
- Poudre de nylon 8 %

### Phase D

- Parfum 0,65 %

### Phase E

Eau qsp 100 %
Conservateur 0.3 %
. Sulfate de magnésium 0,7 % Propylène glycol 3 %

La fabrication de ce fond de teint est identique à celle de l'exemple 1. Le produit obtenu a une belle texture solide et présente les mêmes propriétés que celui de l'exemple 1.

### Exemple 3 : Fond de teint E/H

### Phase A

- Isostéarate de sorbitane (Arlacel 987d'Uniquema) 6 %
- Conservateur 0,2 %

### Phase B

- Uniclear 80 15 %
- Octyl-2-dodécanol 3,6 %
- Iso-paraffine hydrogénée (parléam) 11 %
- Isododécane 22,5 %
- Cyclohexadimethylsiloxane (8 cSt) 5 %

### (DC246 de Dow Corning)

### Phase C

- Dioxyde de titane 4,1 %
- Iso-paraffine hydrogénée 3,3 %
- Oxyde de fer 10,7 %
- Poudre de nylon 4 %

### Phase D

- Parfum 0,65 %

### Phase E

. Eau qsp 100 %
. Conservateur 0,2 %
. Sulfate de magnésium 0,7 %

Le produit obtenu a encore une belle texture solide et présente les mêmes propriétés cosmétiques que celui de l'exemple 1.

### Exemple 4 : Fond de teint E/H

Ce fond de teint se différencie de celui de l'exemple 1 par l'emploi de 17 % d'Uniclear 80 au lieu de 15%. Le produit obtenu est solide et présente une dureté en g de 49,2.

## Revendications

1. Mascara sous forme d'une émulsion solide qui ne s'écoule pas sous son propre poids à température ambiante et pression atmosphérique, ladite émulsion contenant une phase aqueuse et une phase grasse liquide dispersées l'une dans l'autre, la phase aqueuse, la phase grasse liquide et le polymère formant un milieu physiologiquement acceptable,
- la phase grasse liquide étant structurée par au moins un polyamide de masse moléculaire moyenne en poids allant de 1000 à 30 000, comportant a) un squelette polymérique, ayant des motifs répétitifs amide, et b) éventuellement des chaînes grasses pendantes et/ou terminales éventuellement fonctionnalisées, ayant de 12 à 120 atomes de carbone et étant liées à ces motifs amide,
- ladite phase grasse liquide contenant au moins un composé volatile, et
- le mascara comprenant en outre une matière colorante choisie parmi les colorants lipophiles, les colorants hydrophiles, les pigments, les nacres et leurs mélanges.

2. Mascara selon la revendication précédente, **caractérisé en ce que** les chaînes grasses représentent de 40 à 98 % du nombre total des motifs amide et des chaînes grasses.

3. Mascara selon la revendication 1 ou 2, **caractérisé en ce que** les chaînes grasses représentent de 50 à 95 % du nombre total des motifs amide et des chaînes grasses.

4. Mascara selon l'une des revendications 1 à 3, **caractérisé en ce que** les chaînes grasses pendantes sont liées directement à l'un au moins des atomes d'azote des motifs amide.

5. Mascara selon l'une des revendications précédentes, **caractérisé en ce que** la masse molaire moyenne en poids du polyamide va de 2 000 à 20 000 et mieux de 2 000 à 10 000.

6. Mascara selon l'une des revendications précédentes, **caractérisé en ce que** les chaînes grasses terminales sont liées au squelette par des groupes ester.

7. Mascara selon l'une des revendications précédentes, **caractérisé en ce que** les chaînes grasses ont de 12 à 68 atomes de carbone.

8. Mascara selon l'une des revendications 1 à 7, **caractérisé en ce que** le polyamide est choisi parmi les polymères de formule (I) suivante et leurs mélanges : 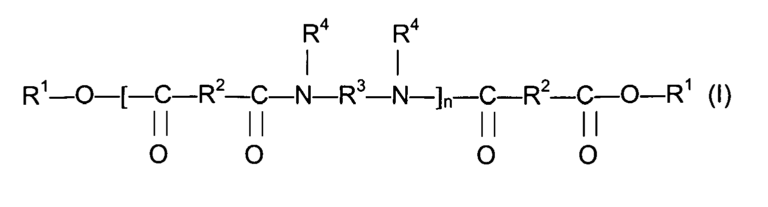 dans laquelle n désigne un nombre de motifs amide tel que le nombre de groupes ester représente de 10 % à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné saturé ou non en C₄ à C₄₂ à condition que 50 % des groupes R² représentent un groupe hydrocarboné saturé ou non en C₃₀ à C₄₂ ; R représente à chaque occurrence indépendamment un groupe organique pourvus d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle saturé ou non en C₁ à C₁₀ ou une liaison directe à R³ ou un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

9. Mascara selon la revendication précédente, **caractérisé en ce que** R¹ est un groupe alkyle en C₁₂ à C₂₂.

10. Mascara selon l'une des revendications 8 ou 9, **caractérisé en ce que** R² sont des groupes ayant de 30 à 42 atomes de carbone.

11. Mascara selon l'une des revendications précédentes, **caractérisé en ce que** le polymère représente de 0,5 à 80 % en matière active du poids total de la composition et mieux de 5 à 40 %.

12. Mascara selon l'une des revendications précédentes, **caractérisé en ce que** la phase grasse liquide contient au moins 40 % du poids total de la phase grasse liquide d'huile apolaire et mieux de 50 à 100 % du poids total de la phase grasse liquide.

13. Mascara selon l'une des revendications précédentes, **caractérisé en ce que** la phase grasse liquide contient au moins une huile hydrocarbonée d'origine minérale, végétale ou synthétique, les esters ou éthers de synthèse, les huiles de silicone et leurs mélanges.

14. Mascara selon l'une des revendications précédentes, **caractérisé en ce que** la phase grasse liquide contient, en outre, au moins une huile non volatile.

15. Mascara selon l'une des revendications précédentes, **caractérisé en ce que** la phase grasse liquide représente de 5 à 98 % du poids total de la composition et mieux de 20 à 75 %.

16. Mascara selon l'une des revendications précédentes, **caractérisé en ce qu'**il se présente sous forme d'émulsion eau-dans-huile.

17. Mascara selon l'une des revendications précédentes, **caractérisé en ce que** la phase aqueuse représente de 1 à 80 % du poids total de la composition et mieux de 5 à 70 %.

18. Mascara selon l'une des revendications précédentes, **caractérisé en ce que** la matière colorante est présente à raison de 0,01 à 50 % du poids total de la composition, de préférence de 5 à 30 %.

19. Mascara selon l'une des revendications précédentes, **caractérisé en ce qu'**il contient en outre un tensioactif ou un mélange de tensio-actifs.

20. Mascara selon l'une des revendications précédentes, **caractérisé en ce que** le tensio-actif est ionique, anionique ou cationique.

21. Mascara selon l'une des revendications précédentes, **caractérisé en ce que** le tensio-actif est choisi parmi
- ceux ayant un HLB inférieur 7 et notamment les esters d'acides gras de polyols comme les mono-, di-, tri- ou sesqui-oléates ou stéarates de sorbitol ou de glycérol, les laurates de glycérol ou de polyéthylène glycol ; les alkyl ou alkoxy diméthicones copolyols à chaîne alkyle ou alkoxy pendante ou en bout de squelette siliconé ayant par exemple de 6 à 22 atomes de carbone, et
- ceux ayant un HLB supérieur à 7 comme les esters d'acides gras de polyéthylène glycol; les esters d'acides gras de sorbitol polyoxyéthylénés ; les alkyl éthers polyoxyéthylénés et les diméthicones copolyols.

22. Mascara selon l'une des revendications précédentes, **caractérisé en ce qu'**il contient au moins un additif choisi parmi les gélifiants de phase aqueuse, les antioxydants, les huiles essentielles, les conservateurs, les parfums, les électrolytes, les charges, les cires, les composés gras pâteux à température ambiante, les neutralisants, les polymères liposolubles ou dispersibles dans le milieu, les actifs cosmétiques ou dermatologiques, les dispersants, et leurs mélanges.

23. Mascara selon l'une des revendications précédentes, **caractérisé en ce qu'**il présente une dureté de 10g à 200g et mieux de 30g à 100g.
